# EUROPÄISCHE PATENTSCHRIFT

(11) **EP 1 072 237 B1**
(45) Veröffentlichungstag und Bekanntmachung des Hinweises auf die Patenterteilung: **09.02.2005**
(21) Anmeldenummer: 00250256.5
(22) Anmeldetag: 27.07.2000
(51) Int. Cl.: A61F 2/36

(54) **Oberschenkelteil einer Hüftendoprothese**
Femoral part of a hip endoprosthesis
Partie fémorale d'une endoprothèse de la hanche

(30) Priorität: 27.07.1999 DE 19935289
(43) Veröffentlichungstag der Anmeldung: 31.01.2001
(73) Patentinhaber: Merete Medical Gmbh, 12247 Berlin (DE)
(72) Erfinder: Kranz, Curt, Dr.-Ing., 14163 Berlin (DE)

(56) Entgegenhaltungen:
- EP-A- 0 159 462
- EP-A- 0 408 109
- EP-A- 0 528 284
- EP-A- 0 668 064
- EP-A- 0 669 116
- DE-A- 3 324 103
- DE-A- 3 811 207
- DE-A- 3 819 948
- DE-A- 3 829 361
- DE-A- 4 421 153
- DE-C- 3 913 874
- DE-U- 8 705 921
- FR-A- 2 686 789

## Beschreibung

Die Erfindung betrifft ein Oberschenkelteil einer Hüftendoprothese, mit einem sich in Längsrichtung zum distalen Ende hin stetig verjüngenden Schaft, der in seinem proximalen Schaftbereich eine ovale Querschnittskontur aufweist, die zum distalen Schaftbereich hin in eine kreisförmige Querschnittskontur übergeht, und mit in Längsrichtung des Schafts verlaufenden Nuten.

Ein derartiges Oberschenkelteil einer Hüftendoprothese ist beispielsweise aus der EP 0 159 462 oder der EP 0 408 109, welche den nächstkommenden Stand der Technik bildet, bekannt, bei der das Nutengesamtprofil, gebildet als Querschnittsfläche aller Nuten, symmetrisch zu einer medial-lateralen Koordinatenfläche ausgebildet ist. Bei diesem bekannten Oberschenkelteil findet daher eine Anpassung des Längs- und des Biegedehnungsverhaltens an die Gegebenheiten des menschlichen Knochens, in dem das Oberschenkelteil verankert wird, nur in dem Maße statt, in dem sich die Querschnittskontur über die Schaftlänge hinweg ändert. Eine erwünschte bessere lokale Anpassung des Elastizitätsverhaltens des Schafts an die Gegebenheiten des menschlichen Knochens, der die Prothese aufnimmt, ist bei dieser bekannten Hüftendoprothese nicht möglich.

Aufgabe der Erfindung ist es, ein Oberschenkelteil der eingangs genannten Art derart weiterzubilden, daß das Elastizitätsverhalten des Schaftes auf die anatornischen Gegebenheiten des menschlichen Femurknochens besser anpaßbar ist.

Diese Aufgabe wird bei dem Oberschenkelteil der eingangs genannten Art erfindungsgemäß durch die Merkmale des Anspruchs 1 gelöst.

Die Vorteile der Erfindung liegen insbesondere darin, daß das Nutengesamtprofil, welches von der jeweiligen Querschnittskontur des Schaftes eingeschlossen ist, im wesentlichen symmetrisch zum Mittelpunkt der jeweiligen Schaft-Querschnittskontur ist. Durch diese spezielle Gestaltung des Nutengesamtprofiles lassen sich die Biegeeigenschaften des Schaftes in gewünschter Weise beeinflussen. Insbesondere läßt sich durch eine geeignete Gestaltung des Nutengesamtprofils die Hauptträgheitsachse des Schaftquerschnitts gegen ein rechtwinkeliges Koordinatensystem, welches aus einer medial-lateralen Koordinatenachse und einer dorsal-ventralen Koordinatenachse gebildet ist, um einen vorgegebenen Winkelversatz verdrehen, um damit eine bessere Anpassung der Dehnungseigenschaften des Schaftes an die anatomischen Gegebenheiten des menschlichen Knochens zu verwirklichen. Die Krafteinleitung in den Knochen, der nach Implantation den Schaft eng umschließt, wird dadurch verbessert, der Knochen wird gleichmäßiger belastet, wodurch eine geringere Resorption des Knochens infolge dieses Streß-Schieldings entsteht.

Gemäß der Erfindung ist das Nutengesamtprofil über die gesamte Schaftlänge punktsymmetrisch zum Mittelpunkt des Schaftquerschnitts ausgebildet, weist aber über die gesamte Schaftlänge hinweg keine gegenüber der medial-lateralen Koordinatenachse und der dorsal-ventralen Koordinatenachse auf. Dies hat zur Folge, daß in dem distalen Schaftbereich, dessen Querschnittskontur kreisförmig ist, die Hauptträgheitsachsen einen konstanten Winkelversatz zur medial-lateralen Koordinatenachse oder Koordinatenebene aufweisen, daß sich also die Biegeeigenschaften über die Länge des distalen Schaftbereichs nicht verändern.

Bevorzugt wird der Winkelversatz der Hauptträgheitsachse dadurch realisiert, daß im lateral-dorsalen Quadranten sowie - wegen der Mittelpunkt-Symmetrie des Nutengesamtprofils - auch im medial/ventralen Quadranten je eine vergleichsweise große Nut vorgesehen ist, die sich im wesentlichen zentral gegen den Mittelpunkt des jeweiligen Schaftquerschnittes erstreckt. Durch diese Materialreduktion in den einander zentral gegenüberliegenden Quadranten wird die Lage der Hauptträgheitsachsen in der gewünschten Weise - im Gegenuhrzeigersinn - gegen die medial-laterale Koordinatenachse herausgedreht. Die im proximalen Schaftbereich sich allmählich ausbildende und zum proximalen Schaftende hin verstärkende Ovalität der Schaft-Querschnittskontur hat zur Folge, daß sich zum proximalen Schaftende hin der Winkelversatz der Hauptträgheitsachse gegenüber der medial-lateralen Koordinatenachse reduziert. Auf diese Weise lassen sich die Biegeeigenschaften im proximalen Schaftbereich relativ zu der Schaft-Querschnittskontur geeignet einstellen. Gemäß einer bevorzugten Ausführungsform der Erfindung enthält das Nutengesamtprofil im lateral/ventralen Quadranten und auch im medial/dorsalen Quadranten der Schaft-Querschnittskontur mehrere Nuten, die im wesentlichen senkrecht zu der medial-lateralen Koordinatenachse ausgerichtet sind und gegenüber den zentral ausgerichteten Nuten eine deutlich kleinere Nutenfläche aufweisen.

Die Nuten weisen bevorzugt einen V-förmigen Querschnitt auf, der sich zu dem Nutengrund stetig verengt und im Nutengrund einen vorgegebenen Rundungsradius aufweist.

Der Nutengrund der zentralen Nuten besitzt vom Mittelpunkt der Querschnittskontur bevorzugt über die ganze Schaftlänge hinweg einen konstanten Abstand. Alternativ nimmt der Abstand des Nutengrunds dieser zentralen Nuten vom Mittelpunkt zum proximalen Schaftende hin stetig zu.

Die in dem lateral/ventralen Quadranten und dem medial/dorsalen Quadranten befindlichen Nuten, welche senkrecht gegen die medial-laterale Koordinatenachse ausgerichtet sind, sind mit ihrem Nutengrund bevorzugt von der medial-lateralen Koordinatenachse bevorzugt unterschiedlich beabstandet, bevorzugt ist jedoch der Abstand des Nutengrundes von dieser medial-lateralen Koordinatenachse über die gesamte Schaftlänge hinweg im wesentlichen konstant.

Die Größe und die Lage der Nuten ist bevorzugt so gewählt, daß die Hauptträgheitsachse HTA im distalen Schaftbereich einen Winkelversatz α aufweist, der etwa 30° bis 40° gegen die medial-laterale Koordinatenachse, und zwar im Gegenuhrzeigersinn, hat. Durch die zum proximalen Schaftende hin stärker werdende ovale Form der Schaft-Querschnittskontur geht dieser Winkelversatz der Hauptträgheitsachse dann auf etwa 10 bis 20° - im Gegenuhrzeigersinn - zurück.

Gemäß einer besonders bevorzugten Ausführungsform der Erfindung läßt sich die ovale Schaft-Querschnittskontur im proximalen Schaftbereich noch mit einer Rotation gegenüber der medial-lateralen Koordinatenebene versehen, um eine bessere Paßgenauigkeit des Schafts in dem Femur-Hohlraum zu realisieren.

Vorteilhafte Weiterbildungen der Erfindung sind durch die Merkmale der Unteransprüche gekennzeichnet.

Im folgenden wird ein Ausführungsbeispiel der Erfindung anhand der Zeichnungen näher erläutert. Es zeigen:
- Fig. 1: eine Seitenansicht eines Oberschenkelteils einer Hüftendoprothese, gesehen von dorsal;
- Fig. 2: eine Ansicht des Oberschenkelteils der Fig. 1, gesehen von lateral; und
- Fig. 3a-d: verschiedene Querschnitte durch den Schaft gemäß den Fig. 1 und 2.

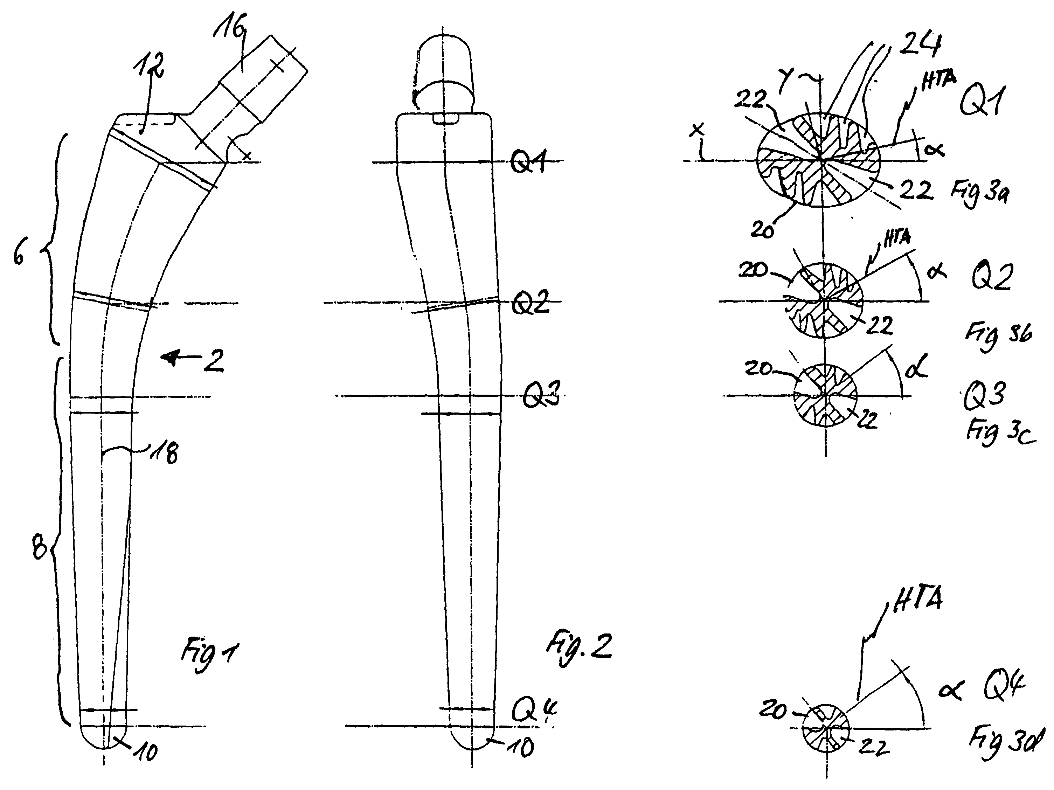

Die Fig. 1 und 2 zeigen ein Oberschenkelteil einer Hüftendoprothese, die einen Schaft 4 besitzt, der sich in Längsrichtung von dem proximalen Schaftende 12 bis zum distalen Schaftende 10 hin erstreckt und sich dabei stetig verjüngt. Am proximalen Schaftende 12 ist über einen Hals 14 ein Konusabschnitt 16 angeformt, auf den ein Kopf mit entsprechender Konusausnehmung aufsetzbar ist. Alternativ ist statt des Konusabschnittes 16 auch ein Kopf einstückig am Schaft angeformt.

Der Schaft 4 besitzt im distalen Schaftbereich 8 eine im wesentlichen gerade verlaufende Längsachse 18, die anschließend im proximalen Schaftbereich 6 gekrümmt verläuft. Von lateral gesehen, Fig. 2, verläuft die Längsachse 18 im distalen Schaftbereich 8 ebenfalls gerade und ist im proximalen Schaftbereich 6 anatomisch gekrümmt. Die Längsachse 18 verläuft jeweils durch den Mittelpunkt der Schaft-Querschnittskontur.

In Fig. 3a bis 3d sind verschiedene Schaftquerschnitte Q1 bis Q4 dargestellt, die in unterschiedlichem Abstand vom proximalen Schaftende 12 durch den Schaft - senkrecht zur Längsachse 18 - hindurch gelegt sind. Der Schaftquerschnitt Q1 befindet sich am proximalen Schaftende 12, der Schaftquerschnitt Q2 befindet sich in der unteren Zone des proximalen Schaftabschnittes 6. Der Schaftquerschnitt Q3 befindet sich in der oberen Zone des distalen Schaftbereichs 8, und der Schaftquerschnitt Q4 befindet sich am distalen Schaftende 10. In jeden Schaftquerschnitt Q1 bis Q4 ist eine medial-laterale Koordinatenachse x und eine senkrecht hierzu verlaufende dorsal-ventrale Koordinatenachse y eingezeichnet, die durch den Mittelpunkt der Schaftquerschnittskontur verlaufen.

Der Schaft besitzt in seinem proximalen Schaftbereich 6 eine ovale Querschnittskontur, die zum distalen Schaftbereich 8 hin allmählich in eine Kreisform übergeht und sich zum distalen Schaftende 6 hin - unter Beibehaltung der Kreisform - verjüngt.

Wie sich insbesondere den Fig. 3a bis 3d entnehmen läßt, ragen in die Querschnittskontur 20 des Schaftes 4 hinein mehrere Nuten 22, 24 und bilden ein Nutengesamtprofil, welches von der Querschnittskontur 20 eingeschlossen wird. Das Nutengesamtprofil besitzt im wesentlichen eine Symmetrie zum Mittelpunkt M der Schaft-Querschnittskontur und verläuft gegenüber der medial-lateralen Koordinatenachse x und der dorsal-ventralen Koordinatenachse y ohne Rotation über die gesamte Länge des Schaftes hinweg. Diese Symmetrie des Nutengesamtprofils zum Mittelpunkt M des Schaftquerschnitts hat zur Folge, daß die Hauptträgheitsachse HTA jeweils als Gerade durch den Mittelpunkt des jeweiligen Schaftquerschnitts Q1...Q4 hindurchläuft. Das Nutengesamtprofil besteht (in der dargestellten Ausführungsform im lateral/dorsalen Quadranten sowie im medial/ventralen Quadranten) aus je einer vergleichsweise großen V-förmigen Nut, die sich im wesentlichen zentral gegen den Mittelpunkt des jeweiligen Schaftquerschnittes erstreckt und sich zu ihrem Nutengrund hin stetig verengt und einen gerundeten Nutengrund besitzt. In den beiden anderen Quadranten, dem lateral/ventralen Quadranten und dem medial/dorsalen Quadranten sind mehrere vergleichsweise kleinere Nuten angeordnet, die im wesentlichen senkrecht zu der medial-lateralen Koordinatenachse x ausgerichtet sind und eine V-förmige Fläche aufweisen, die sich zu ihrem jeweiligen Nutengrund hin stetig verengt. Der Nutengrund der beiden zentralen Nuten 22 besitzt zum Mittelpunkt M hin über die gesamte Schaftlänge hinweg einen konstanten Abstand. Der Nutengrund der restlichen Nuten 24 ist - in der dargestellten Ausführungsform - von Nut zu Nut verschieden, die Abstände sind jedoch über die gesamte Schaftlänge hinweg im wesentlichen konstant. Die Nuten gehen an der Außenkontur des Schaftes abgerundet ineinander über.

Aufgrund der Form und der Größe der Nuten 22, 24 ist die Hauptträgheitsachse HTA im distalen Schaftbereich 8 etwa 37 bis 38° im Gegenuhrzeigersinn gegen die medial-laterale Koordinatenachse x versetzt. Im proximalen Schaftbereich 6 bildet sich dann dieser Winkelversatz der Hauptträgheitsachse zum proximalen Schaftende 12 hin zurück, bis er am proximalen Schaftende 12 nur noch einen Wert von etwa 13° besitzt.

## Patentansprüche

1. Oberschenkelteil einer Hüftendoprothese, mit einem sich in Längsrichtung zum distalen Ende hin stetig verjüngenden Schaft, der in seinem proximalen Schaftbereich eine ovale Querschnittskontur aufweist, die zum distalen Schaftbereich hin in eine kreisförmige Querschnittskontur übergeht, und mit in Längsrichtung des Schafts verlaufenden Nuten,
**dadurch gekennzeichnet,**
**dass** das Nutengesamtprofil, welches von der Querschnittskontur des Schaftes (4) eingeschlossen ist, im wesentlichen symmetrisch zum Mittelpunkt M der jeweiligen Querschnittskontur (Q1 ... Q4) des Schaftes (4) ist und in einem rechtwinkligen Koordinatensystem verläuft, welches aus einer medial-lateralen Koordinatenachse x und einer dorsal-ventralen Koordinatenachse y gebildet ist,
und **dass** das Nutengesamtprofil im lateral/dorsalen Quadranten sowie im medial/ventralen Quadranten der Querschnittskontur (20) je eine Nut (22) aufweist, die sich zentral gegen den Mittelpunkt M des jeweiligen Schaftquerschnittes erstreckt und
**dass** das Nutengesamtprofil im lateral/ventralen Quadranten und im medial/dorsalen Quadranten der Querschnittskontur (20) mehrere Nuten (24) enthält, die im wesentlichen senkrecht zu der medial-lateralen Koordinatenachse X ausgerichtet sind.

2. Oberschenkelteil nach Anspruch 1, **dadurch gekennzeichnet, dass** das Nutengesamtprofil über die gesamte Schaftlänge rotationsfrei in dem rechtwinkligen Koordinatensystem verläuft.

3. Oberschenkelteil nach einem der vorstehenden Ansprüche, **dadurch gekennzeichnet, dass** die Nuten (22, 24) einen V-förmigen Querschnitt aufweisen, der sich zu dem Nutengrund hin stetig verengt und im Nutengrund einen vorgegebenen Krümmungsradius besitzt.

4. Oberschenkelteil nach einem der vorstehenden Ansprüche, **dadurch gekennzeichnet, dass** der Nutengrund der zentralen Nuten (22) vom Mittelpunkt der jeweiligen Schaftquerschnittkontur über die gesamte Schaftlänge hinweg einen konstanten Abstand aufweist.

5. Oberschenkelteil nach einem der Ansprüche 1 bis 3, **dadurch gekennzeichnet, dass** der Nutengrund der zentralen Nuten (22) vom Mittelpunkt M der Schaftquerschnittskontur (20) einen Abstand besitzt, der zum proximalen Schaftende (12) hin zunimmt.

6. Oberschenkelteil nach einem der vorstehenden Ansprüche, **dadurch gekennzeichnet, dass** der Nutengrund im lateral/ventralen Quadranten und im medial/dorsalen Quadranten der Querschnittskontur (20) liegenden einzelnen Nuten (22, 24) von der medial-lateralen Koordinatenachse x einen verschiedenen Abstand aufweist.

7. Oberschenkelteil nach einem der vorstehenden Ansprüche, **dadurch gekennzeichnet, dass** der Abstand des Nutengrundes der einzelnen, im lateral/ventralen Quadranten und im medial/dorsalen Quadranten der Querschnittskontur (20) liegenden einzelnen Nuten (22, 24) ) von der medial-lateralen Koordinatenachse x über die gesamte Schaftlänge hinweg konstant ist.

8. Oberschenkelteil nach einem der vorstehenden Ansprüche, **dadurch gekennzeichnet, dass** die ovale Schaft-Querschnittskontur (20) im proximalen Schaftabschnitt (6) zum proximalen Schaftende (12) hin eine zunehmende Rotation gegenüber einer medial-lateralen Koordinatenachse x erfährt.

9. Oberschenkelteil nach einem der vorstehenden Ansprüche, **dadurch gekennzeichnet, dass** das Nutengesamtprofil so ausgebildet ist, dass die Hauptträgheitsachse HTA des Schaftquerschnitts im distalen Schaftbereich (8) um einen vorgegebenen Winkelversatz α im Gegenuhrzeigersinn gegen die medial-laterale Koordinatenachse x gedreht ist, der zum proximalen Schaftende (12) hin stetig abnimmt.

## Claims

1. Femur section of a hip endoprosthesis having a shaft which tapers continuously towards the distal end in the longitudinal direction, has an oval cross-section contour gradually changing into a circular cross-section and has grooves running along the shaft in a longitudinal direction,
**characterized by**
the overall profile of the grooves which is enclosed by the cross-section contour of the shaft (4) being essentially symmetrical to the respective cross-section contour (Q1 ... Q4) of the shaft (4) and aligned to a rectangular co-ordinate system comprising a medial-lateral co-ordinate axis x and a dorsal-ventral co-ordinate axis y,
and the overall profile of the grooves in the lateral/dorsal quadrant as well as in the medial/ventral quadrant of the cross-section contour (20) having one groove (22) which reaches centrally towards the centre M of the respective shaft cross-section and
the overall profile of the grooves in the lateral /ventral quadrant and in the medial/dorsal quadrant of the cross-section contour (20) comprising multiple grooves (24) which are essentially at right angles to the medial-lateral co-ordinate axis x.

2. Femur section as described in claim 1, **characterized by** the overall profile of the grooves being arranged without a rotary component in the rectangular co-ordinate system along the entire length of the shaft.

3. Femur section as described in one of the preceding claims, **characterized by** the grooves (22, 24) having a v-shaped cross-section which becomes progressively thinner towards the bottom of the groove and having a given radius at the bottom of the groove.

4. Femur section as described in one of the preceding claims, **characterized by** the bottom of the central grooves (22) being at a constant distance from the centre of the respective shaft cross-section contour along the entire length of the shaft.

5. Femur section as described in claims 1 to 3, **characterized by** the distance between the bottom of the central grooves (22) and the centre M of the shaft cross-section contour (20) increasing towards the proximal end (12) of the shaft.

6. Femur section as described in one of the preceding claims, **characterized by** the bottom of the individual grooves (22, 24) in the lateral/ventral quadrant and in the medial/dorsal quadrant of the cross-section contour (20) being at different distances from the medial-lateral co-ordinate axis x.

7. Femur section as described in one of the preceding claims, **characterized by** the distances of the bottom of the individual grooves (22, 24) in the lateral/ventral quadrant and in the medial/dorsal quadrant of the cross-section contour (20) being at a constant distance from the medial-lateral co-ordinate axis x along the entire length of the shaft.

8. Femur section as described in one of the preceding claims, **characterized by** the oval cross-section contour (20) in the proximal section (6) of the shaft being increasingly rotated in relation to the medial-lateral co-ordinate axis x towards the proximal end (12) of the shaft.

9. Femur section as described in one of the preceding claims, **characterized by** the overall profile of the grooves being shaped in such a way that the main axis of inertia, HTA, of the shaft cross-section in the distal section (8) of the shaft is rotated in an anti-clockwise direction with relation to the lateral-medial co-ordinate axis x by a specified angular offset α which decreases uniformly towards the proximal end (12) of the shaft.

## Revendications

1. Partie du fémur d'une prothèse de hanche, avec un corps s'effilant progressivement en direction de l'extrémité distale dans le sens longitudinal, qui possède un profil transversal ovale évoluant en un profil transversal circulaire dans la zone distale, et des rainures longitudinales sur le corps,
**caractérisée par**
le profil global des rainures, enchâssé dans le profil transversal du corps (4), qui est essentiellement symétrique au centre M des sections transversales respectives (Q1 ... Q4) du corps (4) et alignée par rapport à un système de coordonnées rectangulaire composé d'un axe des coordonnées médio-latéral x et d'un axe des coordonnées dorso-ventral y,
et le profil global des rainures dans le quadrant latéral/dorsal et dans le quadrant médian/ventral du profil transversal (20) présentant chacun une rainure (22) qui s'étend le long d'un axe médian vers le centre M de la section du corps respective et
le profil global des rainures dans le quadrant latéral/ventral et dans le quadrant médian/dorsal du profil transversal (20) comprenant plusieurs rainures (24) essentiellement perpendiculaires à l'axe des coordonnées médio-latéral x.

2. Partie du fémur conformément à la revendication 1, **caractérisée par** le profil global des rainures qui s'étend sans composante de rotation sur toute la longueur du corps dans le système de coordonnées rectangulaire.

3. Partie du fémur conformément à l'une des revendications ci-dessus, **caractérisée par** la forme en V de la section transversale des rainures (22, 24) se réduisant progressivement en direction du fond des rainures, et ayant un rayon de courbure déterminé au fond des rainures.

4. Partie du fémur conformément à l'une des revendications ci-dessus, **caractérisée par** le fond des rainures centrales (22) ayant une distance constante sur toute la longueur du corps à partir du centre M du profil transversal du corps.

5. Partie du fémur conformément à l'une des revendications 1 à 3, **caractérisée par** le fond des rainures centrales (22) ayant, à partir du centre M du profil transversal du corps (20), une distance croissante en direction de l'extrémité proximale du corps (12).

6. Partie du fémur conformément à l'une des revendications ci-dessus, **caractérisée par** le fond des rainures individuelles (22, 24) dans le quadrant latéral/ventral et dans le quadrant médian/dorsal du profil transversal (20) étant à des distances différentes de l'axe des coordonnées médio-latéral x.

7. Partie du fémur conformément à l'une des revendications ci-dessus, **caractérisée par** la distance du fond des rainures individuelles (22, 24) dans le quadrant latéral/ventral et dans le quadrant médian/dorsal du profil transversal (20) étant constante sur toute la longueur du corps par rapport à l'axe des coordonnées médio-latéral x.

8. Partie du fémur confdrmément à l'une des revendications ci-dessus, **caractérisée par** le profil transversal ovale du corps (20) dans la zone proximale du corps (6) soumis à une rotation croissante en direction de l'extrémité proximale du corps (12) par rapport à l'axe des coordonnées médio-latéral x.

9. Partie du fémur conformément à l'une des revendications ci-dessus, **caractérisée par** le profil global des rainures conçu de telle manière que l'axe principal d'inertie HTA du profil transversal dans la zone distale du corps (8) est soumis à une rotation dans le sens contraire des aiguilles d'une montre, par rapport à l'axe des coordonnées médio-latéral x, selon un écart angulaire spécifié α se réduisant progressivement en direction de l'extrémité proximale du corps (12).
